# EUROPÄISCHE PATENTSCHRIFT

(11) **EP 2 388 044 B1**
(45) Veröffentlichungstag und Bekanntmachung des Hinweises auf die Patenterteilung: **10.02.2016**
(21) Anmeldenummer: 11165634.4
(22) Anmeldetag: 11.05.2011
(51) Int. Cl.: A61N 1/375, H01R 13/00

(54) **Elektronisches Gerät**
Electronic device
Appareil électronique

(30) Priorität: 21.05.2010 US 346925 P
(43) Veröffentlichungstag der Anmeldung: 23.11.2011
(60) Teilanmeldung: 15199218.7
(73) Patentinhaber: Dyconex AG, 8303 Bassersdorf (CH)
(72) Erfinder: Hauer, Marc, 8050, Zürich (CH); Eck, Stefan, 91315, Höchstadt (DE)
(74) Vertreter: Galander, Marcus

(56) Entgegenhaltungen:
- EP-A2- 0 776 016
- WO-A2-2009/086435
- US-A1- 2005 040 513
- US-A1- 2007 182 364

## Beschreibung

Die Erfindung betrifft ein elektronisches Gerät bzw. elektrisches Bauteil mit einem Gehäuse, einer in dem Gehäuse angeordneten Funktionseinheit, einem die Funktionseinheit nach außen elektrisch anschließenden Anschlussleiter und einer dichten, den Anschlussleiter umgebenden und gegenüber dem Gehäuse isolierenden Durchführung im Gehäuse.

Bei elektronischen Geräten und elektrischen Bauteilen, die besonderen Anforderungen hinsichtlich der dauerhaften Dichtigkeit genügen müssen, sind die sogenannten Durchführungen um einen Anschlussleiter besonders kritische Bereiche. Ihrer Gestaltung wird daher bei solchen Geräten, etwa bei implantierbaren medizin-elektronischem Geräten (Schrittmachern, implantierbaren Kardiovertern, Cochlearimplantaten, implantierbaren Neurostimulatoren etc.) seit Jahren besondere Aufmerksamkeit geschenkt. Technisch etabliert ist dabei die Bildung der Durchführung aus geeigneter Keramik, speziell umgeben von einem metallischen Flansch des Gehäuses.

Speziell für elektrochemische Bauteile, wie etwa Batterien oder Elektrolyt-Kondensatoren, ist aus der WO 2005/001997 A2 eine Durchführung bekannt, bei der ein Führungselement aus einem Polymeren zur Aufnahme des Anschlussleiters vorgesehen ist. Aus der US 2007/0225771 A1 ist eine Durchführungsanordnung bekannt, die beispielsweise in einem Stromquellengehäuse eines implantierbaren medizinischen Geräts Anwendung findet und bei der zwischen dem Anschlussleiter und einem (den volumenmäßig größten Bestandteil der Durchführung bildenden) Keramik- oder Glasring eine dünne Hülse vorgesehen ist, die unter anderem zum Toleranzausgleich bei dem thermisch hoch empfindlichen Metall-Keramik-Aufbau der Durchführung dient. Die dünne Hülse kann insbesondere aus Edelstahl, Aluminium oder Titan bestehen.

Aus der US 5,825,608 ist eine Durchführung eines elektronischen Gerätes bekannt, welche zugleich als Filterkondensator wirkt und beispielsweise bei implantierbaren Geräten, wie etwa Herzschrittmachern, Anwendung finden kann. Bei dieser Durchführung wird ein leitfähiges Polymerharz zur Herstellung elektrischer Verbindungen in einem ansonsten im Wesentlichen aus Keramik aufgebauten Durchführungs-Körper eingesetzt.

Aus der WO 2009/086435 A2 sind Durchführungen bekannt, die Flüssigkristallpolymere enthalten können.

Eine Aufgabe der Erfindung besteht in der Bereitstellung eines verbesserten elektronischen Gerätes bzw. elektrischen Bauteils, welches insbesondere mit hoher Ausbeute hergestellt werden kann, unempfindlich gegenüber thermischen Belastungen ist und einen zuverlässigen Langzeitbetrieb ermöglicht.

Diese Aufgabe wird gemäß einem ersten Aspekt der Erfindung durch ein elektronisches Gerät mit den Merkmalen des Anspruchs 1 gelöst. Zweckmäßige Fortbildungen des Erfindungsgedankens sind Gegenstand der abhängigen Ansprüche.

Die Erfindung schließt den Gedanken ein, die isolierenden Bereiche einer Durchführung des Gerätes bzw. Bauteils aus einem flüssigkristallinen Polymer zu bilden. Hierdurch können insbesondere die bekannten Probleme hinsichtlich der Fertigungstoleranzen und thermischer Belastungen vermieden werden, die bei bekannten Metall-Glas- oder Metall-Keramik-Durchführungskonstruktionen festgestellt wurden.

In einer Ausführung der Erfindung ist das Gerät ausgebildet als implantierbares medizin-elektronisches Gerät. Noch spezieller ist es ausgebildet als Herzstimulationsgerät, insbesondere Herzschrittmacher oder Kardioverter.

Die Durchführung ist allein aus dem flüssigkristallinen Polymeren gebildet, in dem metallische Elemente eingelagert und/oder an dem metallische Elemente angebracht sind.

In Anbetracht des ungünstigeren Diffusionsverhaltens flüssigkristalliner Polymere, verglichen mit jenem von Keramik, sind des Weiteren Ausführungen von Vorteil, bei denen der Diffusionsquerschnitt weitgehend minimiert und/oder die Diffusionslänge weitgehend maximiert ist. Insofern ist eine weitere Ausführung zweckmäßig, bei der die Durchführung alternierend vom Umfang und von einem Mittenbereich ausgehend verlaufende und jeweils nicht vollständig bis zur Mitte bzw. bis zum Umfang reichende Metallschichten aufweist, die auf den Stirnflächen der Durchführung angeordnet und/oder in dem flüssigkristallinen Polymer innerhalb der Durchführung eingebettet sind und im Längsschnitt eine Labyrinthstruktur bilden. Alternativ hierzu kann vorgesehen sein, dass die Durchführung eine spiralig von nahe der oberen Stirnfläche zu nahe der unteren Stirnfläche verlaufende Metallschicht aufweist. Auch sonstige konstruktive Ausgestaltungen, die dem Ziel eines verringertem Diffusionsquerschnitts bzw. einer erhöhten Diffusionslänge dienen, sind als vorteilhaft anzusehen.

Eine weitere Ausführung der Erfindung sieht vor, dass mindestens eine Metallschicht, insbesondere eine Mehrzahl von Metallschichten einer Labyrinthstruktur, mit dem Anschlussleiter fest verbunden und/oder integral mit diesem gebildet ist.

Das elektrische Bauteil gemäß dem zweiten Aspekt der Erfindung ist insbesondere als Kondensator ausgebildet oder als Sende- und/oder Baugruppe eine Nachrichtenübertragungs- oder Filteranordnung. Auch sonstige Bauteile, bei denen es auf eine hochgradig dichte und zugleich thermisch stabile Durchführung ankommt, können vorteilhaft den vorgeschlagenen Aufbau aufweisen.

Im Übrigen kann die Durchführung des elektrischen Bauteils auf die gleiche Weise ausgestaltet sein wie bei dem elektronischen Gerät gemäß dem ersten Aspekt der Erfindung. Speziell bei den erwähnten konstruktiven Ausgestaltungen, bei denen der LCP mit metallischen Elementen (speziell Schichten) kombiniert ist, lassen sich mit der Durchführung zugleich elektrische Zusatzfunktionen realisieren.

Insbesondere können die metallischen Elemente zusammen mit Abschnitten des flüssigkristallinen Polymers eine Kapazität bilden, welche derart angeordnet und an die Funktionseinheit des Gerätes angeschlossen ist, dass hiermit eine elektrische Funktion, insbesondere eine Filter- bzw. frequenzselektive Abschirmfunktion, des Gerätes realisiert wird. Z. B. der weiter oben erwähnte Aufbau der Durchführung aus alternierenden LCP- und Metall-Lagen ermöglicht neben der bereits erwähnten Kondensator- sowie Filter-Funktion auch weitere elektrische/elektronische Funktionen speziell in Verbindung mit einem "Daughterboard", das z.B. durch eine flexible Verbindung mit der Durchführung kombiniert ist und direkt im Bereich der Gehäuseöffnung liegt. Bei geeigneter Dimensionierung lassen sich hierdurch insbesondere äußere HF-Störfelder abschirmen und die elektromagnetische Störsicherheit bzw. Verträglichkeit (EMI / EMV) eines entsprechenden Geräts erhöhen.

Die Verbindung der vorgeschlagenen Durchführung mit der Wandung eines Metallgehäuses, speziell Ti-Gehäuses, sollte so gestaltet sein, dass ein Einschweißen in das Gehäuse möglich ist - was eine geeignete Materialauswahl, Geometrie und gegebenenfalls das Vorsehen einer Schweißschutzblende erfordert -, oder die Durchführung wird direkt durch Kleben oder Löten in ein Gehäuseteil (eine Halbschale) eingefügt, insbesondere bevor das Gehäuse zusammengesetzt wird.

Vorteile und Zweckmäßigkeiten der Erfindung ergeben sich im Übrigen aus der nachfolgenden Beschreibung zweier Ausführungsbeispiele anhand der Figuren. Von diesen zeigen:
- Fig. 1A und 1B: eine schematische Längsschnittdarstellung sowie schematische perspektivische Darstellung einer ersten Ausführungsform der Erfindung,
- Fig. 2: eine schematische Längsschnittdarstellung einer zweiten Ausführungsform der Erfindung und
- Fig. 3: eine schematische Längsschnittdarstellung einer dritten Ausführungsform der Erfindung.

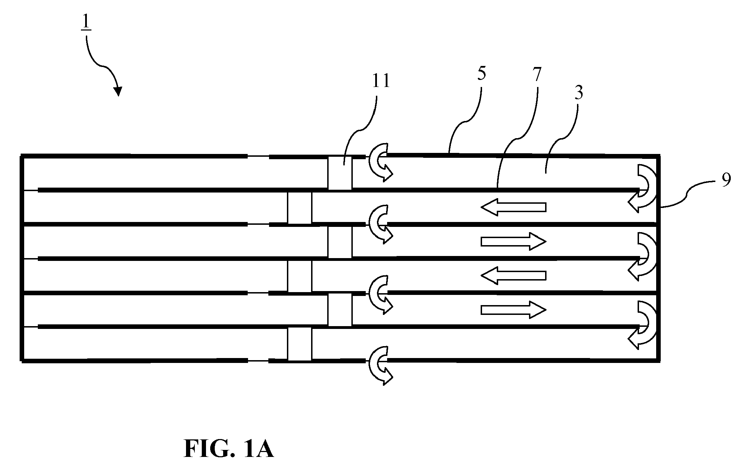

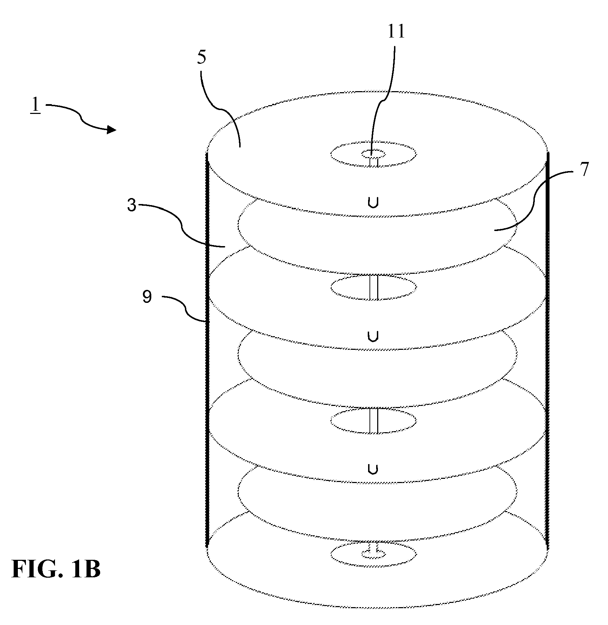

Fig. 1A und 1B zeigen schematisch das Wirkprinzip und den grundsätzlichen Aufbau einer Durchführung 1, die allein aus einem flüssigkristallinen Polymeren 3 mit außen angebrachten bzw. eingelagerten ersten und zweiten Metallschichten 5 und 7 aufgebaut ist. Jeweils eine der ersten Metallschichten 5 ist auf den beiden Stirnflächen der im Wesentlichen zylinderförmigen Durchführung 1 angeordnet, und bei der hier dargestellten Ausführung sind zwei weitere der ersten Metallschichten in die Durchführung, parallel zu den Stirnflächen, eingelagert. Die ersten Metallschichten sind jeweils mit einer leitfähigen Mantelschicht 9 der Durchführung kontaktiert und reichen jeweils nicht ganz bis an einen zentralen Leiter (Anschlussleiter) 11 heran. Dem gegenüber sind die jeweils zwischen den ersten Metallschichten 5 in den Durchführungs-Körper eingelagerten zweiten Metallschichten 7 leitend mit dem Anschlussleiter 11 verbunden, reichen aber nicht ganz bis an den Mantel-Leiter 9 heran.

Wie mit den Pfeilen in Fig. 1A symbolisiert, wird dadurch ein labyrinthartiger Aufbau des LCP-Grundkörpers mit erheblich verlängerten Diffusionswegen und verringerten Diffusionsquerschnitten geschaffen.

Der Aufbau nach Fig. 1A und 1B mit den alternierend angeschlossenen Metallschichten 5, 7 bildet einen Kondensator mit den beiden über den leitfähigen Mantel 9 und den Innenleiter 11 realisierten Anschlüssen, dessen Kapazität durch die geeignete Dimensionierung auf einen Wert eingestellt werden kann, der eine Zusatzfunktion im Zusammenwirken mit einer im elektronischen Gerät vorgesehenen Funktionseinheit, etwa eine Ausfilterung äußerer elektromagnetischer Störungen durch ein geeignet abgestimmtes Filter ermöglicht.

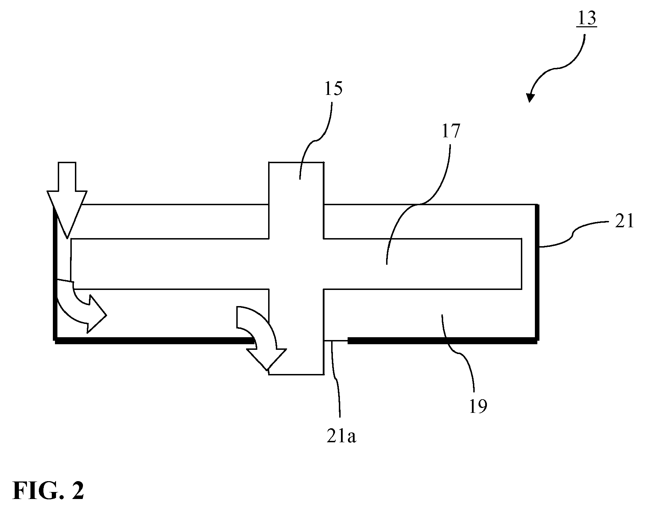

Ein ähnlicher Effekt, wenn auch weniger ausgeprägt, wird mit dem vereinfachten Aufbau einer weiteren Durchführung 13 gemäß Fig. 2 erreicht. Hier ist um einen zentralen Leiter (Anschlussleiter) 15 herum und in elektrischem Kontakt mit diesem eine Metallscheibe 17 angeordnet, die mit einem flüssigkristallinen Polymeren 19 derart umspritzt ist, dass die Stirnflächen und der Umfang der Scheibe 17 vollständig in den Polymeren eingeschlossen sind. Das hierdurch gewonnene Teil ist in einen Topf 21 aus leitfähigem Material mit einer zentrischen Öffnung 21a eingesetzt, deren Durchmesser größer als derjenige des Anschlussleiters 15 ist. Hierdurch ist für eine elektrische Isolation zwischen Anschlussleiter 15 und Topf 21 gesorgt, und für diffundierende Ionen ergibt sich der wiederum mit Pfeilen bezeichnete, gegenüber einem Durchführungs-Aufbau ohne die Scheibe 17 verlängerte Diffusionsweg und verringerte Diffusionsquerschnitt.

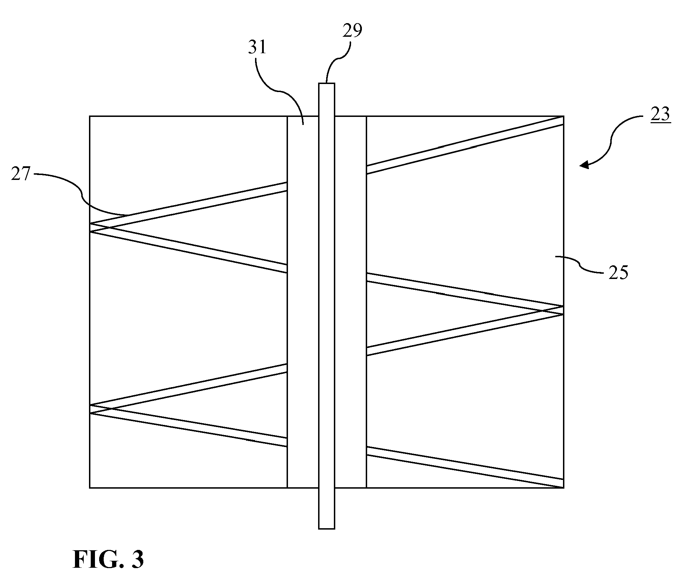

Fig. 3 zeigt schematisch einen weiteren möglichen Aufbau am Beispiel einer Durchführung 23, bei der in einen zylindrischen Grundkörper 25 aus einem flüssigkristallinen Polymeren eine spiralig angeordnete Metallfolie 27 eingebettet ist. In einer zentralen Öffnung dieses Durchführungs-Körpers liegt ein Anschlussleiter 29 mit einer Keramikhülle 31. Es handelt sich hier also um einen materialseitig hybriden Durchführungs-Aufbau aus LCP und Keramik.

## Patentansprüche

1. Elektronisches Gerät mit einem Gehäuse, einer in dem Gehäuse angeordneten Funktionseinheit, einem die Funktionseinheit nach außen elektrisch anschließenden Anschlussleiter und einer dichten, den Anschlussleiter umgebenden und gegenüber dem Gehäuse isolierenden Durchführung (1, 13, 25) im Gehäuse, wobei die Durchführung allein aus einem flüssigkristallinen Polymer (3, 19, 25) gebildet ist, in dem metallische Elemente (5, 7, 9, 11, 15, 17, 21, 27, 29) eingelagert und/oder au dem metallische Elemente angebracht sind.

2. Gerät nach Anspruch 1, ausgebildet als implantierbares medizin-elektronisches Gerät.

3. Gerät nach Anspruch 2, ausgebildet als Herzstimulationsgerät, insbesondere Herzschrittmacher oder Kardioverter.

4. Gerät nach Anspruch 1, wobei die Durchführung (1) zylinderförmig ist und dadurch eine obere Stirnfläche und eine untere Stirnfläche besitzt und wobei die Durchführung alternierend vom Umfang und von einem Mittenbereich ausgehend verlaufende und jeweils nicht vollständig bis zur Mitte bzw. bis zum Umfang reichende Metallschichten (5, 7) aufweist, die auf den Stirnflächen der Durchführung angeordnet und/oder in dem flüssigkristallinen Polymer innerhalb der Durchführung eingebettet sind und im Längsschnitt eine Labyrinthstruktur bilden, wodurch ein verlängerter Diffusionsweg und ein verringerter Diffusionsquerschnitt für Moleküle bzw. Ionen aus dem Inneren des Geräts nach Außen bestimmt wird.

5. Gerät nach Anspruch 1, wobei die Durchführung (1) zylinderförmig ist und dadurch eine obere Stirnfläche und eine untere Stirnfläche besitzt und wobei eine spiralig von nahe der oberen Stirnfläche zu nahe der unteren Stirnfläche verlaufende Metallschicht (27) aufweist, die einen verlängerten Diffusionsweg und verringerten Diffusionsquerschnitt für Moleküle bzw. Ionen aus dem Inneren des Gerätes nach Außen bestimmt.

6. Gerät nach einem der Ansprüche 1 bis 5, wobei die metallischen Elemente (5, 7, 9, 11, 15, 17, 21, 27, 29) zusammen mit Abschnitten des flüssigkristallinen Polymers (3, 19, 25) eine Kapazität bilden, welche derart angeordnet und an die Funktionseinheit des Gerätes angeschlossen ist, dass hiermit eine elektrische Funktion, insbesondere eine Filterfunktion, des Gerätes realisiert wird.

7. Gerät nach einem der Ansprüche 1 bis 6, wobei mindestens eine Metallschicht (5, 7, 17, 27), insbesondere eine Mehrzahl von Metallschichten einer Labyrinthstruktur, mit dem Anschlussleiter (11, 15, 29) fest verbunden und/oder integral mit diesem gebildet ist.

## Claims

1. An electronic device comprising a housing, a functional unit disposed in the housing, a connecting lead electrically connecting the functional unit outwardly, and a sealed feedthrough (1, 13, 25) in the housing, the feedthrough surrounding the connecting lead and insulating it with respect to the housing, wherein the feedthrough is formed solely from a liquid crystal polymer (3, 19, 25), in which metallic elements (5, 7, 9, 11, 15, 17, 21, 27, 29) are incorporated and/or to which metallic elements are attached.

2. The device according to Claim 1, formed as an implantable medical electronic device.

3. The device according to Claim 2, formed as a cardiac stimulation device, in particular a cardiac pacemaker or cardioverter.

4. The device according to Claim 1, wherein the feedthrough (1) is cylindrical and therefore has an upper end face and a lower end face, and wherein the feedthrough comprises metal layers (5, 7), which extend in an alternating manner starting from the circumference and from a centre region and do not fully reach to the centre and to the circumference respectively, and which are disposed on the end faces of the feedthrough and/or are embedded in the liquid crystal polymer inside the feedthrough and form a labyrinth structure in the longitudinal section, whereby an elongated diffusion path and a reduced diffusion cross section for molecules and/or ions from the inside of the device to the outside is determined.

5. The device according to Claim 1, wherein the feedthrough (1) is cylindrical and thus has an upper end face and a lower end face, and wherein the feedthrough has a metal layer (27) extending helically from close to the upper end face to close to the lower end face, which metal layer determines an extended diffusion path and reduced diffusion cross section for molecules and/or ions from the inside of the device to the outside.

6. The device according to one of Claims 1 to 5, wherein the metallic elements (5, 7, 9, 11, 15, 17, 21, 27, 29) together with portions of the liquid crystal polymer (3, 19, 25) form a capacitor, which is disposed and is connected to the functional unit of the device in such a way that an electrical function of the device, in particular a filter function, is implemented in this way.

7. The device according to one of Claims 1 to 6, wherein at least one metal layer (5, 7, 17, 27), in particular a plurality of metal layers of a labyrinth structure, is fixedly connected to the connecting lead (11, 15, 29) and/or is formed integrally therewith.

## Revendications

1. Appareil électronique avec un boîtier, une unité fonctionnelle disposée dans le boîtier, un conducteur de connexion électrique raccordant l'unité fonctionnelle vers l'extérieur et une traversée (1, 13, 25) dans le boîtier, étanche, entourant le conducteur de connexion et l'isolant vis-à-vis du boîtier, où la traversée seule est formée par un polymère à cristaux liquides (3, 19, 25), dans lequel sont noyés des éléments de métal (5, 7, 9, 11, 15, 17, 21, 27, 29) et/ou sur lequel sont apposés des éléments métalliques.

2. Appareil selon la revendication 1, conçu en tant qu'appareil électronique médical implantable.

3. Appareil selon la revendication 2, conçu en tant qu'appareil de stimulation cardiaque, notamment en tant que stimulateur cardiaque ou défibrillateur cardiaque.

4. Appareil selon la revendication 1, dans lequel la traversée (1) est de forme cylindrique et de ce fait, possède une surface frontale supérieure et une surface frontale inférieure, et dans lequel la traversée présente des couches de métal (5, 7) s'étendant en partant alternativement du pourtour et d'une zone centrale et n'atteignant non complètement ni respectivement le centre, ni respectivement le pourtour, qui sont disposées sur les surfaces frontales de la traversée et/ou enfouies dans le polymère à cristaux liquides à l'intérieur de la traversée, et qui forment en coupe longitudinale une structure de labyrinthe, ce par quoi un chemin de diffusion rallongé et une section transversale de diffusion diminuée pour les molécules, respectivement, les ions, à partir de l'intérieur de l'appareil vers l'extérieur se trouvent déterminés.

5. Appareil selon la revendication 1, dans lequel la traversée (1) est de forme cylindrique et de ce fait, possède une surface frontale supérieure et une surface frontale inférieure, et dans lequel se présente une couche de métal (27) s'étendant en forme d'une spirale à partir d'à peu près la surface frontale supérieure jusqu'à peu près la surface frontale inférieure, qui détermine un chemin de diffusion rallongé et une section transversale de diffusion diminuée pour les molécules, respectivement, les ions, à partir de l'intérieur de l'appareil vers l'extérieur.

6. Appareil selon l'une des revendications 1 à 5, dans lequel les éléments de métal (5, 7, 9, 11, 15, 17, 21, 27, 29) forment un condensateur, ensemble avec des segments de polymère à cristaux liquides (3, 19, 25), lequel est disposé de telle manière et est raccordé à l'unité fonctionnelle de l'appareil que, par ce moyen, une fonction électrique, notamment une fonction de filtre de l'appareil se trouve réalisée.

7. Appareil selon l'une des revendications 1 à 6, dans lequel au moins une couche de métal (5, 7, 17,27), notamment une multiplicité de couches de métal d'une structure en labyrinthe, sont solidement reliées avec le conducteur de connexion (11, 15, 29) et/ou sont formées intégralement avec celui-ci.
